# EUROPEAN PATENT SPECIFICATION

(11) **EP 3 545 761 B1**
(45) Date of publication and mention of the grant of the patent: **13.10.2021**
(21) Application number: 19164424.4
(22) Date of filing: 21.03.2019
(51) Int. Cl.: A01N 1/02, A61F 2/00, A61F 2/14

(54) **MEDICAL DEVICE FOR PRESERVING EXPLANTED CORNEAS**
MEDIZINISCHE VORRICHTUNG ZUR KONSERVIERUNG VON EXPLANTIERTEN KORNEAS
DISPOSITIF MÉDICAL POUR CONSERVER LES CORNÉES EXPLANTÉS

(30) Priority: 30.03.2018 IT 201800004148
(43) Date of publication of application: 02.10.2019
(73) Proprietor: AL.CHI.MI.A. S.r.l., 35020 Ponte San Nicolò (PD) (IT)
(72) Inventor: Beccaro, Mauro, 35010 Cadoneghe (Padova) (IT); Bettini, Enrico, 30032 Fiesso D'Artico (Venezia) (IT); Signori, Paolo, 37100 Verona (IT)
(74) Representative: Ponchiroli, Simone

(56) References cited:
- EP-A1- 0 014 319
- WO-A1-2006/052478
- US-A1- 2006 113 207
- US-A1- 2008 294 149

## Description

The present invention relates to a medical device for preserving explanted corneas and, more particularly, to a medical device comprising a viewing chamber; i.e. a container specially designed to contain a cornea immersed in preserving liquid, which at the same time allows the cornea to be viewed from outside the container using special analysis equipment.

In particular, in the known way, a conventional viewing chamber comprises a vessel having a base wall, a lateral wall and an upper opening, which inside it forms a preserving chamber, inside of which a supporting unit for a corneoscleral disc has in turn been placed. A lid can then be removably screwed onto, and therefore joined to, the vessel. The lid is also generally designed to cooperate with the supporting unit to hold the corneoscleral disc in the correct position on the supporting unit.

In turn, the supporting unit is formed by a plurality of bars perpendicular to the base wall, which are distributed along a circle coaxial to a central axis of the vessel (corresponding to the lid screwing axis). The corneoscleral disc rests on the tops of the bars and the circle has a large enough diameter for the tops of the bars to be in contact with the scleral part of the disc.

At the supporting unit, the base wall and/or the lid (advantageously, both) on one hand profile inwards towards the corneoscleral disc, whereas on the other hand they have respective portions made of a material transparent to visible light. These transparent portions, which are brought towards the corneoscleral disc, allow visual examinations of the cornea from the outside of the container.

An example of a traditional viewing chamber is described in US Patent Application 2008/294149. WO 2006/052478 A1 also discloses an apparatus for preservation and transportation of an explanted cornea. Although the prior art technology works well with regard to preserving and viewing the cornea, there have been further demands placed by users over time.

In particular, potential issues have been raised regarding the possibility of ensuring the security of the entire procedure beginning with the explantation of the cornea until the cornea is subsequently reimplanted and, in particular, the procedures for transferring corneas between the place of explantation and the eye banks, or between the eye banks and the places of subsequent reimplantation.

Indeed, guaranteeing the security of these procedures means being able to guarantee that the medical device in which the cornea is preserved is properly closed and guaranteeing that the device, once closed, has not been re-opened, that is to say, that the cornea has not subsequently been subjected to further handling which could have contaminated it. Indeed, the main aim of viewing chambers is to allow all necessary checks to be carried out on the cornea from the outside of the viewing chamber only.

In this context, the technical purpose of the present invention is to produce a medical device for preserving explanted corneas that offers a solution to the issues mentioned above.

In particular, the technical purpose of the present invention is to produce a medical device for preserving explanted corneas that allows greater security to be ensured over the entire procedure, commencing with the explantation of the cornea and until the time of its subsequent reimplantation, or over part of that procedure, than current prior art devices and methods allow.

The technical purpose and the aims indicated above are substantially achieved by a medical device for preserving explanted corneas in accordance with the appended claims. The present invention provides a medical device for preserving explanted corneas as defined in claim 1. Preferred embodiments are defined in the dependent claims.

Further features and the advantages of the present invention will become more apparent after a careful reading of the detailed description below of several preferred, non-limiting embodiments of a medical device for preserving explanted corneas, with reference to the accompanying drawings, in which:

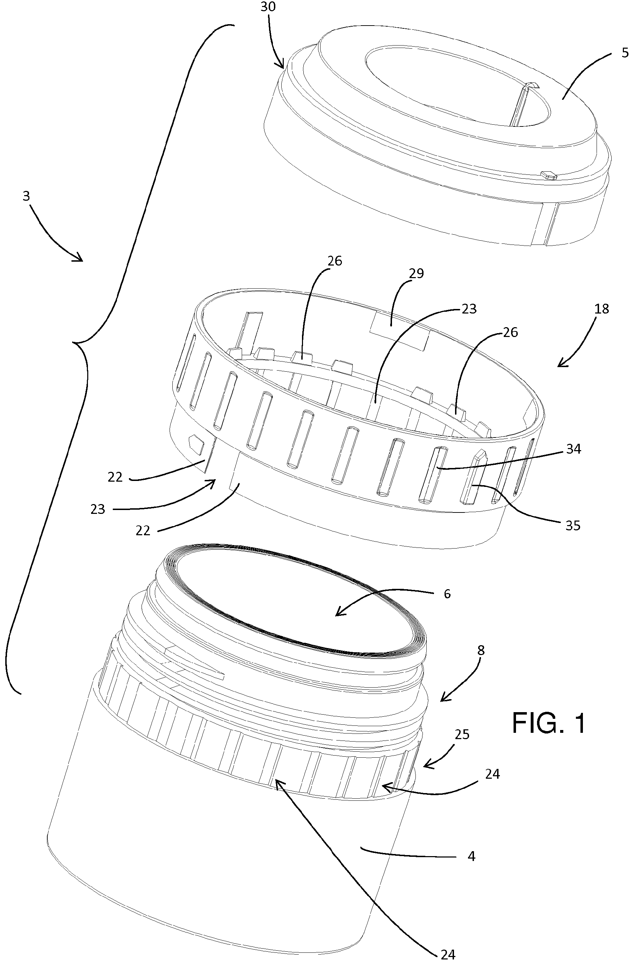

- Figure 1 is an exploded axonometric view of several elements of a medical device in accordance with the present invention;
- Figure 2 is a cross-section in an axial section plane of the elements of Figure 1;

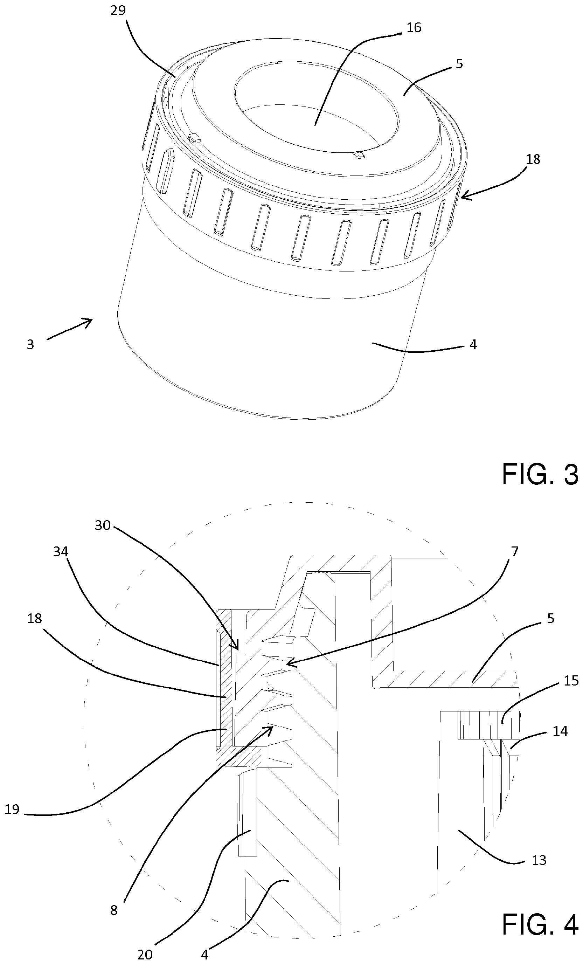

- Figure 3 is an assembled view of the elements of Figure 1;
- Figure 4 is a detail of the elements of Figure 3, cross-sectioned according to the axial section plane of Figure 2;
- Figure 5 is a schematic axonometric view of a first embodiment of a medical device in accordance with this invention;
- Figure 6 is a schematic axonometric view of a second embodiment of a medical device in accordance with this invention;
- Figure 7 is a top view of a locking ring of Figure 1, with some parts transparent;
- Figure 8 shows the locking ring of Figure 7, cross-sectioned along line VIII - VIII;
- Figure 9 shows the locking ring of Figure 7, cross-sectioned along line IX - IX;
- Figure 10 shows the locking ring of Figure 7, cross-sectioned along line X - X;
- Figure 11 is an axonometric top view of the locking ring of Figure 7;
- Figure 12 is an axonometric bottom view of the locking ring of Figure 7;

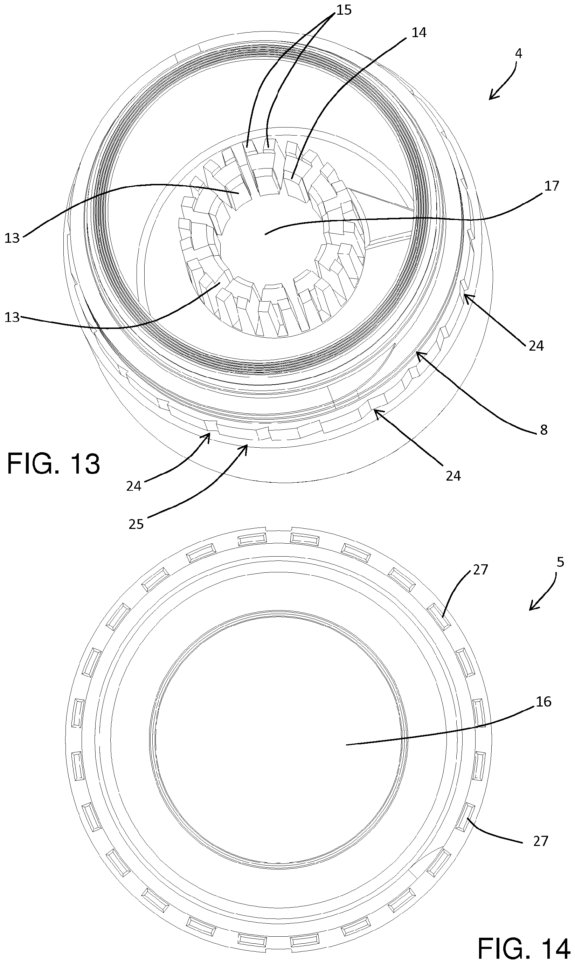

- Figure 13 is an axonometric top view of a vessel of Figure 1;
- Figure 14 is a bottom view of a lid of Figure 1;

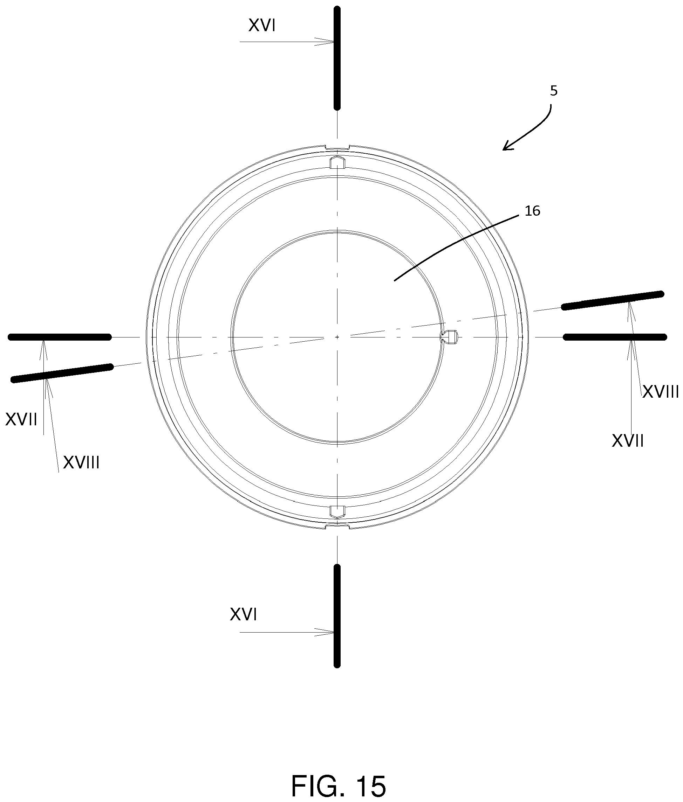

- Figure 15 is a top view of the lid of Figure 14;

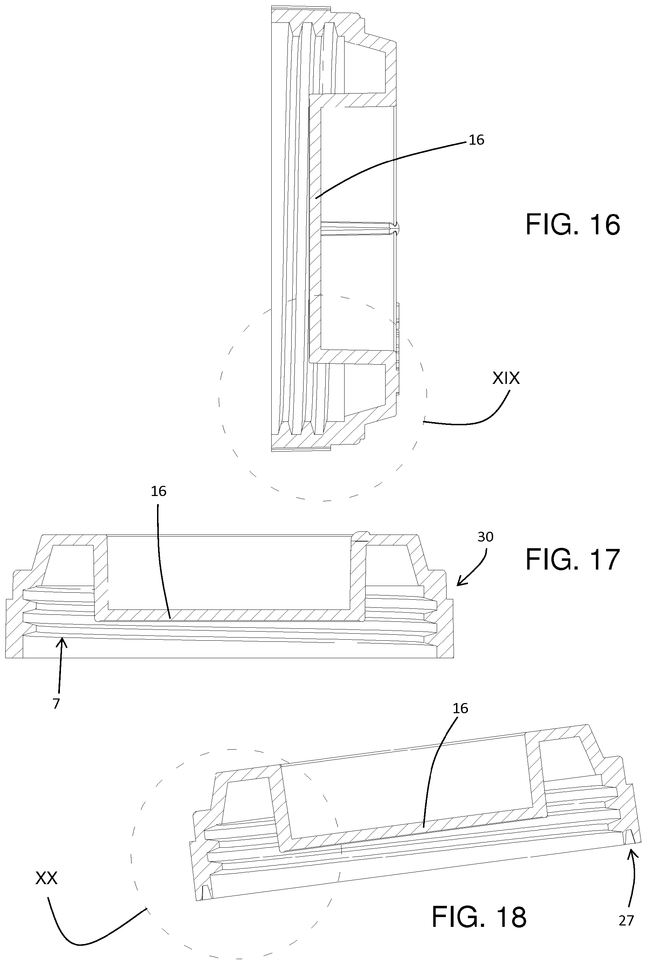

- Figure 16 shows the lid of Figure 15, cross-sectioned along line XVI - XVI;
- Figure 17 shows the lid of Figure 15, cross-sectioned along line XVII - XVII;
- Figure 18 shows the lid of Figure 15, cross-sectioned along line XVIII - XVIII;
- Figure 19 is an enlargement of detail XIX of Figure 16; and
- Figure 20 is an enlargement of detail XX of Figure 18.

Referring to the drawings above, a complete medical device for preserving explanted corneas in accordance with this invention has been assigned reference number 1.

In general, the medical device according to this invention comprises a sealed outer main case 2, which contains a container 3 for preserving and viewing a cornea (that is to say, a viewing chamber). Everything contained inside the outer main case 2 is preferably sterile.

As with prior art viewing chambers, the container 3 according to the present invention comprises a vessel 4 and a first lid 5 that is removably joinable or joined to the vessel 4 for closing an upper opening 6 of it. Advantageously, the first lid 5 is joined or joinable to the vessel 4 by screwing and, to this end, it has a (preferably inner) first thread 7 that is joinable to a corresponding (preferably outer) second thread 8 fixed to the vessel 4 (in particular, to its lateral wall 9).

Indeed, the vessel 4 has a base wall 10 and a lateral wall 9 which extends from a perimetric area of the base wall 10 and whose opposite end delimits the upper opening 6. On its inside, the vessel 4 delimits a preserving chamber 11, which is intended, in use, to receive the liquid-immersed cornea for its preservation.

A supporting unit 12 for a corneoscleral disc, which can take any form according to requirements, is also positioned inside the preserving chamber 11. However, in the embodiment shown, the supporting unit 12 comprises a plurality of bars, which extend perpendicularly to the base wall 10 (towards the upper opening 6) and which are distributed along a circle coaxial to a central axis of the vessel 4 (the axis corresponds to the axis of screwing of the lid to the vessel 4, and the lateral wall 9 is cylindrical in shape).

Each bar 13 forms two resting areas 14, 15 for the corneoscleral disc: an inner area 14 (upward-facing and on which the disc actually rests) and two outer areas 15 (centre-facing and against which the rim of the corneoscleral disc can rest). Moreover, the circumference on which the bars 13 rest has a large enough diameter for the resting areas 14, 15 to be in contact, in use, with the scleral part of the disc rather than the corneal part.

Furthermore, advantageously, the first lid 5 is intended to cooperate with the supporting unit 12 to hold the corneoscleral disc in the correct position on the supporting unit 12. This is particularly achieved by reducing the distance between a central portion 16 of the first lid 5 and the bars 13 of the supporting unit 12 when the first lid 5 is joined to the vessel 4.

Preferably, to allow better viewing of the cornea through the container 3, at the supporting unit 12, the base wall 10 and the first lid 5 on one hand profile inwards towards the corneoscleral disc, whereas on the other hand they have respective central portions 16, 17 (coaxial to the central axis of the vessel 4 and sufficiently wide to allow the entire cornea to be observed on a corneoscleral disc resting on the supporting unit 12) made from a material transparent to visible light and with a smooth surface finish. Advantageously, the material transparent to visible light is polymer-based, the polymer being selected from the group comprising polycarbonate, polystyrene and polymethyl methacrylate, all optical-grade. This definition refers to an optically transparent material, that is to say, a material capable of allowing much of the light incident on its surface to be transmitted without reflection; in general, this behaviour is ensured by the absence of structural defects (cavities, cracks, etc.) and by the lack of absorption by the molecules of these materials.

Preferably, however, all of the material (or materials) which the vessel 4 and the first lid 5 are made of is sterilisable by means of gamma ray sterilisation and/or by ethylene oxide sterilisation. The use of polymethyl methacrylate with added substances capable of rendering it sterilisable by gamma rays is particularly preferred.

Advantageously, in the preferred embodiment, the vessel 4 and the supporting unit 12 are formed by a single piece of moulded plastic material, as is the first lid 5 (which may or may not be made of the same material).

According to the embodiments, inside of the outer main case 2, the container 3 may be either open (that is to say, with the first lid 5 separate from the vessel 4) or closed (that is to say, with the first lid 5 joined to the vessel 4). Where the container 3 is closed, it can also be designed to be empty or to contain preserving liquid for corneas (the liquid is not shown in the accompanying figures). It should be noted that, in the context of the present invention, "preserving liquid" means any liquid in which a cornea can be immersed for a reasonably long period of time during any phase, from the moment the cornea is explanted from a deceased patient to the moment it is reimplanted in a living patient; therefore, the definition also includes liquids for processing the cornea, such as liquids to reduce bacterial load, deturgescence liquids, etc.

In accordance with the present invention, the medical device 1 is ready for use, in the sense that it is (internally, at least) sterile and therefore, once removed from the outer main case 2, a corneoscleral disc can be immediately inserted into it; as mentioned above, according to the embodiments, the preserving liquid may already be present (preferred solution) or must be added by the operator.

In accordance with the present invention, the medical device 1 also comprises a first locking ring 18 which is joinable or already joined to both the first lid 5 and to the vessel 4, outside them. The function of the first locking ring 18, once attached to the vessel 4 and to the first lid 5 when the container 3 is closed, is to prevent removal of the first lid 5 from the vessel 4 unless the first locking ring 18 is broken and/or deformed. Indeed, the first locking ring 18 is made to be breakable or deformable through a simple user-operated mechanical action, advantageously, by pulling the first lid 5 off the vessel 4.

Consequently, in accordance with this invention, when the first lid 5 is joined to the vessel 4 and the first locking ring 18 is joined both to the first lid 5 and to the vessel 4, two situations can occur. As long as the first locking ring 18 remains in an intact and non-deformed condition, removal of the first lid 5 from the vessel 4 is prevented. To be able to remove the first lid 5 from the vessel 4, the first locking ring 18 must adopt the broken or deformed condition, thus "releasing" the first lid 5.

In greater detail, although other technically equivalent solutions are possible, in the embodiment shown, the first locking ring 18 comprises a first annular portion 19 and a second annular portion 20, which are side by side (in a direction parallel to the central axis) and connected to each other by a facilitated breaking portion 21.

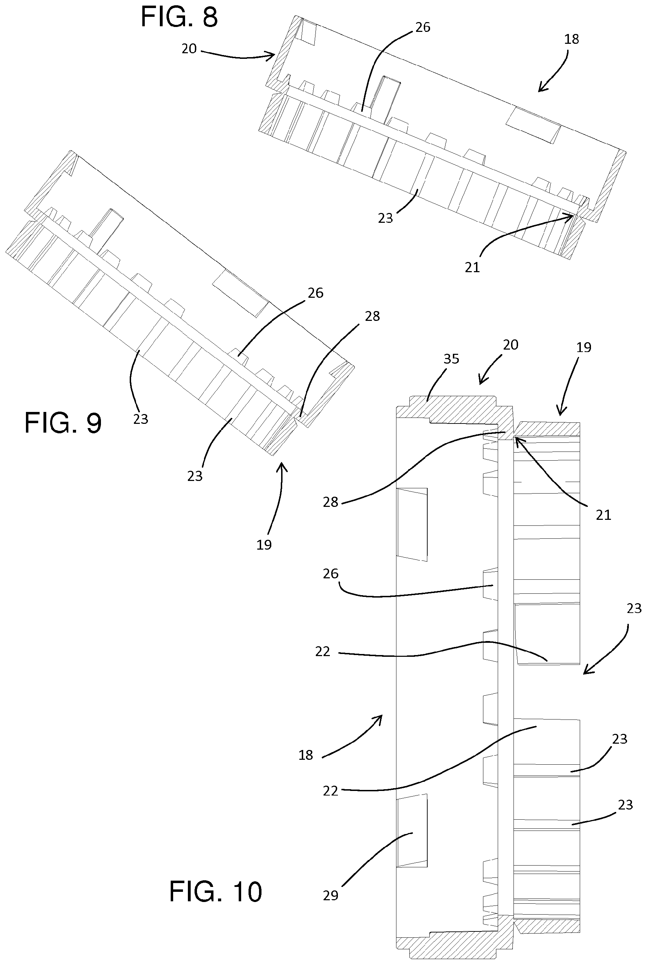

As shown in Figures 8 to 10, the facilitated breaking portion 21 is preferably a portion having limited thickness that can be torn when sufficient force or torque is applied between the first annular portion 19 and the second annular portion 20 (in the preferred embodiment, wherein the overall diameter of the first locking ring 18 is approximately 50 mm, the thickness of the facilitated breaking portion 21 is between 0.2 mm and 0.8 mm, and preferably is equal to 0.5 mm).

The first annular portion 19, which has a width (diameter) smaller than the second annular portion 20, is designed to interact with the vessel 4, while the second annular portion 20 is designed to interact with the first lid 5.

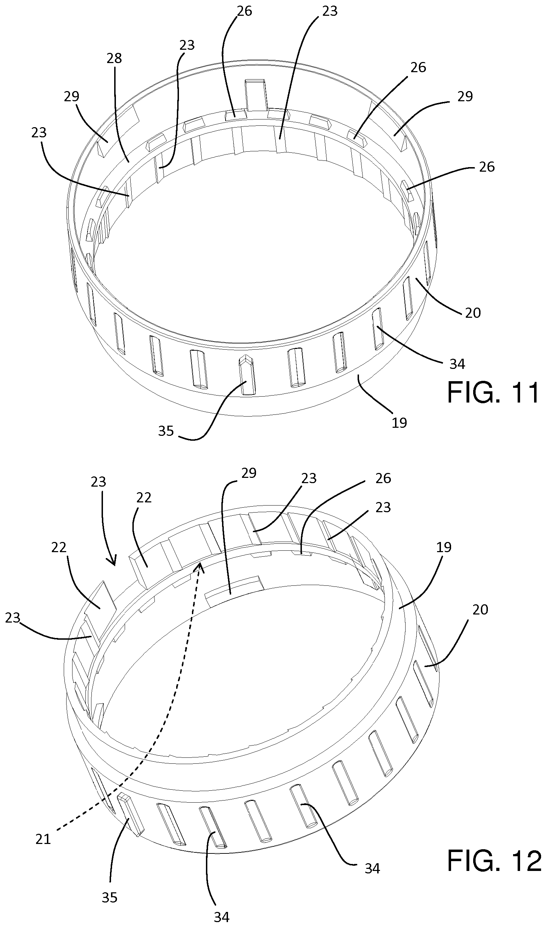

Moreover, while the second annular portion 20 is formed by a closed ring, the first annular portion 19 is preferably formed by an open ring having two ends 22 separated by a predetermined gap 23 (Figures 10 and 12), so that it can be manually torn off the second annular portion 20 (the user can detach the first annular portion 19 from the second annular portion 20 starting from either of the two ends 22 by gradually rotating it around the container 3). To allow a better grip on the second annular portion 20 while removing the first annular portion 19, the outer part of the second annular portion 20 may be fitted with recesses 34 and/or ridges 35.

In the embodiment shown, the first annular portion 19 comprises first engaging elements suitable for interacting with second engaging elements made on the vessel 4, for preventing a rotation of the first annular portion 19 relative to the vessel 4 in a direction that corresponds to the direction of unscrewing of the lid from the vessel 4, instead allowing rotation in the opposite direction.

Advantageously, one of either the first engaging elements or the second engaging elements are constituted of first projecting teeth 23 having a saw-tooth cross-section, and the other of corresponding first seats 24 shaped to match the first projecting teeth 23. In the embodiment shown, the first projecting teeth 23 are fixed to the inside of the first annular portion 19, whereas the first seats 24 are made at an indent 25 in the lateral wall 9 of container 3, placed near the second thread 8. Both the first projecting teeth 23 and the first seats 24 extend radially relative to the central axis of screwing of the first lid 5 to the vessel 4.

In turn, the second annular portion 20 comprises third engaging elements suitable for interacting with fourth engaging elements made on the first lid 5, for preventing the disengagement of the second annular portion 20 from the first lid 5, once the first locking ring 18 has been joined to the first lid 5.

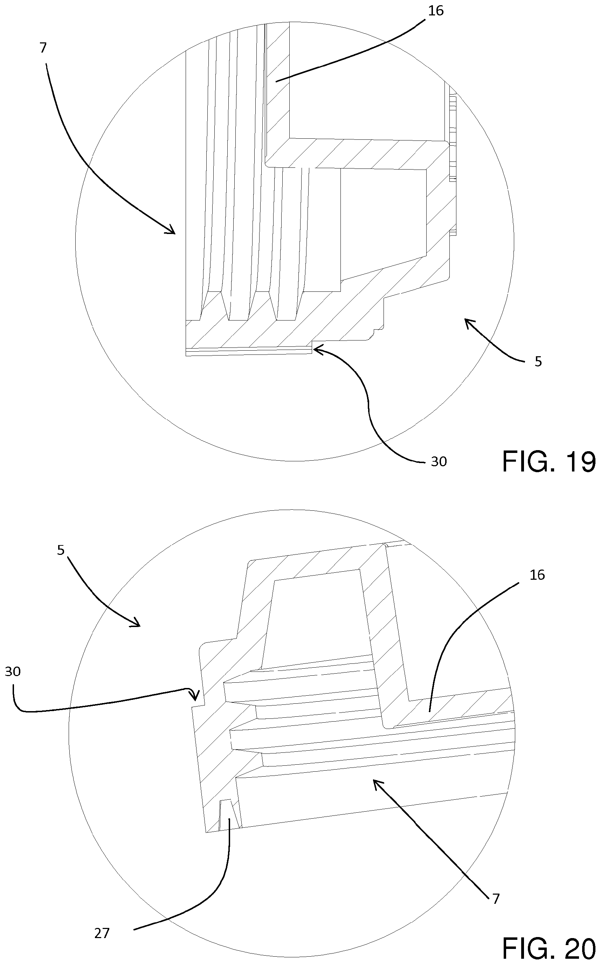

According to the preferred embodiment, one of either the third engaging elements or the fourth engaging elements are constituted of second projecting teeth 26, and the other of corresponding second seats 27 shaped to match the second projecting teeth 26. Advantageously, both the second projecting teeth 26 and the second seats 27 extend parallel to the central axis of screwing of the first lid 5 to the vessel 4. As shown in Figures 8 to 11, in the embodiment shown, the second projecting teeth 26 are fixed to an inner shoulder 28 of the second annular portion 20, indenting in towards the central axis, whereas the second seats 27 are made in a lower edge of the first lid 5 (Figure 20).

The interaction between the second projecting teeth 26 and the second seats 27 prevents the first lid 5 from rotating relative to the second annular portion 20 of the first locking element.

Preferably, however, the second annular portion 20 also comprises third projecting teeth 29, with a saw-tooth profile, projecting inwards towards the central axis and suitable for interacting with a second shoulder 30 made on the outside of the first lid 5, to prevent the disengagement of the first lid 5 from the second annular portion 20.

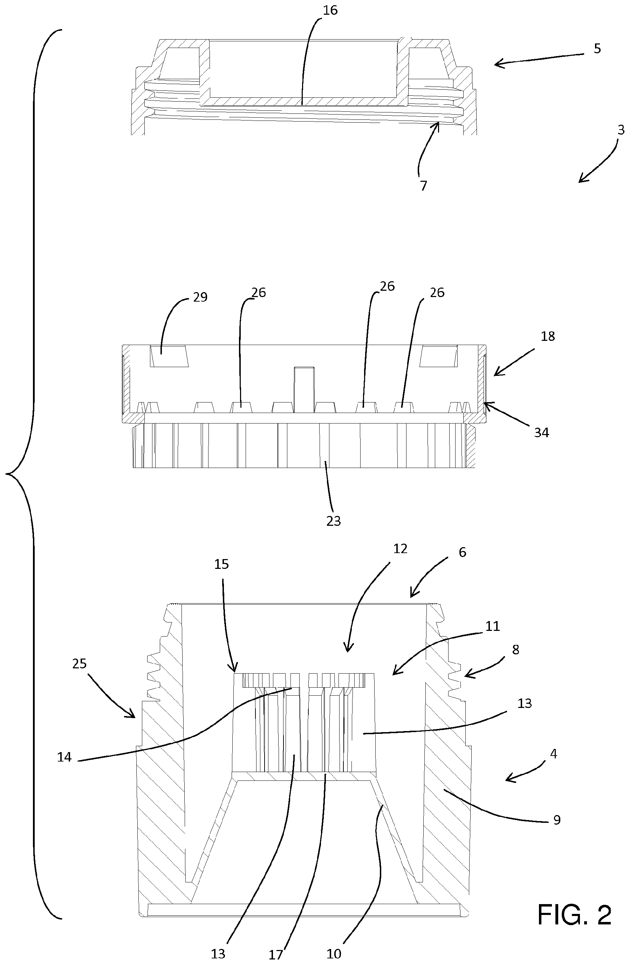

This configuration means that the first locking ring 18 can be attached to the first lid 5 with a simple axial movement (from the bottom upwards in Figure 2) and the consequent snap insertion occurring at the third projecting teeth 29 before the latter is joined to the vessel 4; the container 3 can then be closed and the first lid 5 can be locked simply by screwing the first lid 5 onto the vessel 4 completely. Indeed, the saw-tooth shape of the first projecting teeth 23 allows them to slide onto the lateral wall 9 of the vessel 4 unimpeded during screwing; once screwed on, the interaction between the first projecting teeth 23 and the first seats 24 prevents the first lid 5 (which is fixed to the first locking ring 18) from being unscrewed.

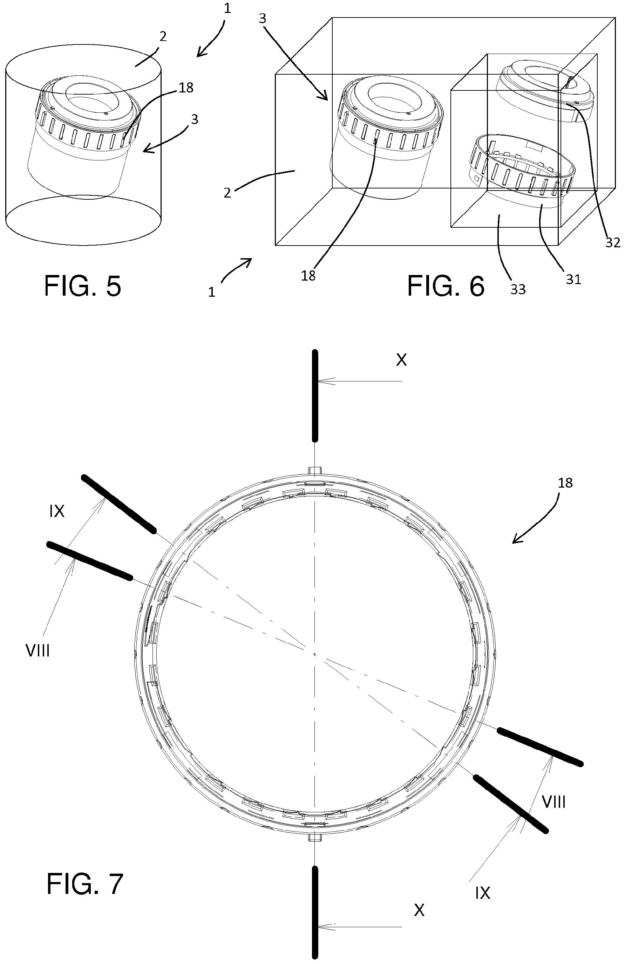

As mentioned above, in its simplest embodiment, the present invention only comprises the outer main case 2, the container 3 (open, closed but empty, or closed with preserving liquid inside) and the first locking ring 18. The latter, according to this embodiment, is preferably only attached to the first lid 5 when the container 3 is open, but other options are also available (for example, in Figure 5 where the container 3 is closed and the first locking ring 18 is attached to the first lid 5).

Things may vary slightly in more complex embodiments. In one embodiment of the invention, the (intact and non-deformed) first locking ring 18 is joined both to the first lid 5 and to the vessel 4, to prevent them from being separated, but a second locking ring 31, joinable to the vessel 4 but at this point separate from the first lid 5 and the vessel 4, is also present inside the outer main case 2. Advantageously, the second locking ring 31 is identical to the first locking ring 18 and may be used to replace the first locking ring 18 when this becomes broken or deformed following the opening of the previously closed container 3 (in this case, the container 3 will preferably contain the preserving liquid).

In addition to a second locking ring, a further - more complex - embodiment is designed so that a second lid 32 may also be joinable both to the vessel 4 and to the second locking ring 31. In this case, the second locking ring 31 does not need to be identical to the first locking ring, but it is nevertheless advisable that at least the respective first projecting teeth 23 are identical, thus allowing it to interact with the vessel 4. Likewise, the second lid 32 does not need to be identical to the first, but needs only to be usable in a similar way in order to close the vessel 4. In contrast, the second lid 32 and the second locking ring 31 must be mutually compatible so as to be able to interact in exactly the same way as indicated for the first lid 5 and the first locking ring 18. In the preferred embodiment, however, the second lid 32 and the second locking ring 31 are identical to the first lid and locking ring.

In this embodiment, the second lid 32 and the second locking ring 31 are intended, in use, to substitute the first lid 5 and the first locking ring 18 once the container 3 has been opened by the first locking ring 18 being broken or deformed, without having to set aside the first locking ring 18 and the first lid 5, which can simply be thrown away.

In the preferred embodiment, as shown in Figure 6, the second locking ring 31 and the second lid 32, if present, are sterile and inserted into an inner secondary case 33, which is in turn inserted into the outer main case 2.

The operation and use of the device according to the present invention can be inferred from the structural description above. Nevertheless, the operating methods of a couple of the preferred embodiments will be briefly outlined below, while the operating methods of all other embodiments are easily understandable.

In all cases, the user first opens the outer main case 2 and extracts the sterile container 3.

If the container is open, the user fills it with a preserving liquid, whereafter the user places a corneoscleral disc on the resting areas 14, 15. If the first locking ring 18 is not joined to the first lid 5 at this point, the user first joins them to each other and then screws the first lid 5 onto the container 3. Once screwed on, the intact first locking ring 18, now in place, assures the person handling the same container 3 at a later stage that the container 3 has never been opened following the insertion of the cornea. It is worth noting that, if the container 3 is initially open, the device will, understandably, not comprise the second lid 32 and the second locking ring 31.

If, on the other hand, the container 3 is closed and contains the liquid, advantageously the first locking ring 18 will be joined to the first lid 5 to assure the user that the container 3 is correctly sealed before use. The user will then open the container 3 by breaking or deforming the first locking ring 18 and unscrewing the first lid 5. If the second lid 32 and the second locking ring 31 are available, the first lid 5 and the first locking ring 18 will be thrown away once the container 3 has been opened.

After having placed a corneoscleral disc on the resting areas 14, 15, in such a way that it is immersed in the preserving liquid, the user will take the second lid 32 and the second locking ring 31, opening the inner secondary case 33 if necessary, and will close the container 3 again according to the same procedures as described above for the use of the first lid 5 and the first locking ring 18.

The present invention offers significant advantages.

The present invention has made it possible to produce of a medical device for preserving explanted corneas that allows greater security to be guaranteed over the entire procedure, commencing with the explantation of the cornea and until the time of its subsequent reimplantation, or over part of that procedure, than the prior art devices allow. The present invention enables users to receive a clear indication of the fact that the viewing chamber has never been opened during a given phase of the procedure, for instance after the cornea has been inserted into it.

Furthermore, the present invention has made it possible to refine a medical device for preserving explanted corneas that is ready for use and that, at least in the most complete embodiments, provides the user with everything necessary for securely preserving corneas.

Finally, it is worth noting that the present invention is relatively easy to make and that the cost associated with its implementation is also not very high.

Many modifications and variations can be made to the embodiments described above without departing from the scope of the invention as defined in the appended claims.

Any materials, shapes and dimensions of the various components may vary according to requirements.

## Claims

1. A medical device for preserving explanted corneas, comprising:
a sealed outer main case (2);
a container (3), for preserving and viewing a cornea, contained in the sealed outer main case (2);
wherein the container (3) in turn comprises:
a vessel (4) having a base wall (10), a lateral wall (9) and an upper opening (6) and which inside it forms a preserving chamber (11);
a supporting unit (12) for a corneoscleral disc positioned in the preserving chamber (11); and
a first lid (5) removably joinable or joined to the vessel (4) for closing the upper opening (6);
wherein the medical device (1) is ready for use and also comprises a first locking ring (18) which is joinable or joined to both the first lid (5) and to the vessel (4), outside them, and is breakable or deformable; wherein when the first lid (5) is joined to the vessel (4) and the first locking ring (18) is joined both to the first lid (5) and to the vessel (4):
in an intact and non-deformed condition, the first locking ring (18) prevents removal of the first lid (5) from the vessel (4); and
in a broken or deformed condition, the first locking ring (18) allows removal of the first lid (5) from the vessel (4);
and wherein either:
- the first lid (5) is joined to the vessel (4); and the first locking ring (18) is intact and non-deformed, and is joined both to the first lid (5) and to the vessel (4) for preventing them from being separated; and wherein the medical device (1) also comprises a second locking ring (31) joinable to the vessel (4), also inserted in the outer case and separate from the first lid (5) and from the vessel (4); or
- the first locking ring (18) is separate both from the first lid (5) and from the vessel (4) for, in use, being joined to the vessel (4) and to the lid (5) after a cornea has been inserted in the vessel (4).

2. The medical device according to claim 1, comprising the second locking ring (31) and also comprising a liquid for preserving corneas in the preserving chamber (11).

3. The medical device according to claim 1 or 2, comprising the second locking ring (31) and also comprising a second lid (32) joinable both to the vessel (4) and to the second locking ring (31), the second lid (32) and the second locking ring (31) being intended, in use, for substituting the first lid (5) and the first locking ring (18) once the container (3) has been opened and a cornea has been inserted in it.

4. The medical device according to any of the preceding claims, wherein the first lid (5) is joined or joinable to the vessel (4) by screwing onto it.

5. The medical device according to claim 4, wherein the first locking ring (18) and/or the second locking ring (31) comprises a first annular portion (19) and a second annular portion (20), which are axially side by side and connected to each other by a facilitated breaking portion (21), the first annular portion (19) comprising first engaging elements suitable for interacting with second engaging elements made on the vessel (4) for preventing a rotation of the first annular portion (19) relative to the vessel (4) in a direction that corresponds to a direction of unscrewing of the lid from the vessel (4), and the second annular portion (20) comprising third engaging elements suitable for interacting with fourth engaging elements made on the lid for preventing disengagement of the second annular portion (20) from the lid once the first locking ring (18) has been joined to the lid.

6. The medical device according to claim 5, wherein one of either the first engaging elements or the second engaging elements are constituted of first projecting teeth (23) having a saw-tooth cross-section, and the other of corresponding first seats (24) shaped to match the projecting teeth, the first projecting teeth (23) extending radially relative to a central axis of screwing of the first lid (5) to the vessel (4).

7. The medical device according to claim 5 or 6, wherein one of either the third engaging elements or the fourth engaging elements are constituted of second projecting teeth (26) extending parallel to a central axis of screwing of the first lid (5) to the vessel (4), and the other of corresponding second seats (27) shaped to match the projecting teeth.

8. The medical device according to any of the preceding claims, wherein everything that is present inside the outer main case (2) is sterile.

## Patentansprüche

1. Eine medizinische Vorrichtung zur Konservierung von explantierten Korneae, Folgendes umfassend: ein versiegeltes, äußeres Hauptgehäuse (2);
einen Behälter (3) zur Konservierung und zur Ansicht einer Kornea, die im versiegelten äußeren Hauptgehäuse (2) enthalten ist;
wobei der Behälter (3) seinerseits Folgendes umfasst:
ein Gefäß (4), das eine Bodenwand (10), eine Seitenwand (9) und eine obere Öffnung (6) hat und das in seinem Inneren eine Konservierungskammer (11) bildet;
eine Halterungseinheit (12) für eine korneosklerale Scheibe, die in der Konservierungskammer (11) positioniert ist; und
einen ersten Deckel (5), der verbindbar oder verbunden mit dem Gefäß (4) entfernt werden kann, um die obere Öffnung (6) zu schließen;
wobei die medizinische Vorrichtung (1) gebrauchsfertig ist und außerdem einen ersten Sicherungsring (18) beinhaltet, der verbindbar oder verbunden sowohl mit dem ersten Deckel (5) als auch mit dem Gefäß (4) ist, sich außerhalb von diesen befindet und der sich brechen lässt oder verformbar ist;
wobei der erste Deckel (5) mit dem Gefäß (4) verbunden ist und der erste Sicherungsring (18) sowohl mit dem ersten Deckel (5) als auch mit dem Gefäß (4) verbunden ist:
in einem intakten und nicht verformtem Zustand der erste Sicherungsring (18) das Entfernen des ersten Deckels (5) vom Gefäß (4) verhindert; und
in einem gebrochenen oder verformten Zustand der erste Sicherungsring (18) das Entfernen des ersten Deckels (5) vom Gefäß (4) erlaubt;
und wobei entweder:
- der erste Deckel (5) mit dem Gefäß (4) verbunden ist; und der erste Sicherungsring (18) intakt und nicht verformt ist und sowohl mit dem ersten Deckel (5) als auch mit dem Gefäß (4) verbunden ist, um zu verhindern, dass sie getrennt werden; und wobei die medizinische Vorrichtung (1) außerdem einen zweiten Sicherungsring (31) beinhaltet, der mit dem Gefäß (4) verbunden werden kann, der ebenfalls in das äußere Gehäuse eingefügt und vom ersten Deckel (5) und vom Gefäß (4) getrennt ist; oder
- der erste Sicherungsring (18) sowohl vom ersten Deckel (5) als auch vom Gefäß (4) getrennt ist, um im Gebrauch mit dem Gefäß (4) und mit dem Deckel (5) verbunden zu werden, nachdem eine Kornea in das Gefäß (4) eingefügt wurde.

2. Die medizinische Vorrichtung nach dem Patentanspruch 1, den zweiten Sicherungsring (31) beinhaltend und außerdem eine Flüssigkeit zur Konservierung von Korneae in der Konservierungskammer (11) beinhaltend.

3. Die medizinische Vorrichtung nach den Patentansprüchen 1 oder 2, den zweiten Sicherungsring (31) beinhaltend und außerdem einen zweiten Deckel (32) beinhaltend, der sowohl mit dem Gefäß (4) als auch mit dem zweiten Sicherungsring (31) verbunden werden kann, der zweite Deckel (32) und der zweite Sicherungsring (31) sind dabei im Gebrauch vorgesehen, um den ersten Deckel (5) und den ersten Sicherungsring (18) zu ersetzen, sobald der Behälter (3) geöffnet wurde und eine Kornea darin eingefügt wurde.

4. Die medizinische Vorrichtung nach jedem der vorigen Patentansprüche, wobei der erste Deckel (5) mit dem Gefäß (4) verbunden oder verbindbar ist, indem er daraufgeschraubt wird.

5. Die medizinische Vorrichtung nach dem Patentanspruch 4, wobei der erste Sicherungsring (18) und/oder der zweite Sicherungsring (31) einen ersten ringförmigen Abschnitt (19) und einen zweiten ringförmigen Abschnitt (20) beinhalten, die axial nebeneinander liegen und durch einen Sollbruchabschnitt (21) miteinander verbunden sind, der erste ringförmige Abschnitt (19) beinhaltet dabei erste Eingriffselemente, die sich zur Interaktion mit zweiten Eingriffselementen, die am Gefäß (4) hergestellt sind, eignen, um eine Drehung des ersten ringförmigen Abschnitts (19) im Verhältnis zum Gefäß (4) in eine Richtung zu verhindern, die einer Richtung des Abschraubens des Deckels vom Gefäß (4) entspricht, und der zweite ringförmige Abschnitt (20) dabei dritte Eingriffslemente beinhaltet, die sich dazu eignen, mit vierten Eingriffselementen zu interagieren, die im Deckel hergestellt sind, um die Trennung des zweiten ringförmigen Abschnitts (20) vom Deckel zu verhindern, sobald der erste Sicherungsring (18) mit dem Deckel verbunden wurde.

6. Die medizinische Vorrichtung nach dem Patentanspruch 5, wobei entweder die ersten Eingriffselemente oder die zweiten Eingriffselemente aus ersten hervorstehenden Zähnen (23) bestehen, die einen Sägezahn-Querschnitt haben, und die anderen aus entsprechenden ersten Sitzen (24), die so geformt sind, dass sie zu den hervorstehenden Zähnen passen, die ersten hervorstehenden Zähne (23) erstrecken sich dabei radial im Verhältnis zu einer zentralen Achse des Anschraubens des ersten Deckels (5) an das Gefäß (4).

7. Die medizinische Vorrichtung nach dem Patentanspruch 5 oder 6, wobei entweder die dritten Eingriffselemente oder die vierten Eingriffselemente aus zweiten hervorstehenden Zähnen (26) bestehen, die sich parallel zu einer zentralen Achse des Anschraubens des ersten Deckels (5) an das Gefäß (4) erstrecken, und die anderen aus entsprechenden zweiten Sitzen (27), die so geformt sind, dass sie zu den hervorstehenden Zähnen passen.

8. Die medizinische Vorrichtung nach jedem der vorigen Patentansprüche, wobei alles, was sich innerhalb des äußeren Hauptgehäuses (2) befindet, steril ist.

## Revendications

1. Un dispositif médical pour la conservation de cornées explantées, comprenant :
un boîtier principal extérieur (2) scellé ;
un contenant (3), pour la conservation et la visualisation d'une cornée, contenu dans le boîtier principal extérieur (2) scellé ;
dans lequel le contenant (3) comprend à son tour :
un récipient (4) ayant une paroi de base (10), une paroi latérale (9) et une ouverture supérieure (6) et qui définit intérieurement une chambre de conservation (11);
une unité de support (12) pour un disque cornéoscléral, positionnée dans la chambre de conservation (11) ; et
un premier couvercle (5) pouvant être associé ou est associé de façon amovible au récipient (4) pour fermer l'ouverture supérieure (6) ;
dans lequel le dispositif médical (1) est prêt à l'emploi et comprend également une première bague de blocage (18) qui peut être associée ou est associée à la fois au premier couvercle (5) et au récipient (4), à l'extérieur de ceux-ci, et qui est rupturable ou déformable ;
dans lequel lorsque le premier couvercle (5) est associé au récipient (4) et que la première bague de blocage (18) est associée à la fois au premier couvercle (5) et au récipient (4) :
dans une condition intacte et non déformée, la première bague de blocage (18) empêche le retrait du première couvercle (5) du récipient (4) ; et
dans une condition rompue ou déformée, la première bague de blocage (18) permet le retrait du premier couvercle (5) du récipient (4) ;
et dans lequel, soit :
- le premier couvercle (5) est associé au récipient (4) ; et la première bague de blocage (18) est intacte et non déformée, et est associée à la fois au premier couvercle (5) et au récipient (4) pour les empêcher d'être séparés ; et dans lequel le dispositif médical (1) comprend aussi une deuxième bague de blocage (31) pouvant être associée au récipient (4), insérée elle aussi dans le boîtier extérieur et séparée du premier couvercle (5) et du récipient (4) ; soit
- la première bague de blocage (18) est séparée à la fois du premier couvercle (5) et du récipient (4) pour, en utilisation, être associée au récipient (4) et au couvercle (5) une fois qu'une cornée a été insérée dans le récipient (4).

2. Le dispositif médical selon la revendication 1, comprenant la deuxième bague de blocage (31) et comprenant également un liquide pour la conservation de cornées dans la chambre de conservation (11).

3. Le dispositif médical selon la revendication 1 ou 2, comprenant la deuxième bague de blocage (31) et comprenant également un deuxième couvercle (32) pouvant être associé à la fois au récipient (4) et à la deuxième bague de blocage (31), le deuxième couvercle (32) et la deuxième bague de blocage (31) étant destinés, en utilisation, à remplacer le premier couvercle (5) et la première bague de blocage (18) une fois que le contenant (3) a été ouvert et qu'une cornée a été insérée à l'intérieur.

4. Le dispositif médical selon l'une quelconque des revendications précédentes, dans lequel le premier couvercle (5) est associé ou peut être associé au récipient (4) par vissage sur celui-ci.

5. Le dispositif médical selon la revendication 4, dans lequel la première bague de blocage (18) et/ou la deuxième bague de blocage (31) comprend une première portion annulaire (19) et une deuxième portion annulaire (20), qui sont axialement côte à côte et raccordées l'une à l'autre par une portion à rupture facilitée (21), la première portion annulaire (19) comprenant des premiers éléments d'assujettissement destinés à interagir avec des deuxièmes éléments d'assujettissement réalisés sur le récipient (4) pour empêcher une rotation de la première portion annulaire (19) par rapport au récipient (4) dans une direction qui correspond à une direction de dévissage du couvercle du récipient (4), et la deuxième portion annulaire (20) comprenant des troisièmes éléments d'assujettissement destinés à interagir avec des quatrièmes éléments d'assujettissement réalisés sur le couvercle pour empêcher le désengagement de la deuxième portion annulaire (20) du couvercle une fois que la première bague de blocage (18) a été associée au couvercle.

6. Le dispositif médical selon la revendication 5, dans lequel les premiers éléments d'assujettissement ou les deuxièmes éléments d'assujettissement sont constitués les uns par des premières dents en saillie (23) ayant une section en dent de scie, et les autres par des premiers logements (24) correspondants conformés pour correspondre aux dents en saillie, les premières dents en saillie (23) s'étendant radialement par rapport à un axe central de vissage du premier couvercle (5) au récipient (4).

7. Le dispositif médical selon la revendication 5 ou 6, dans lequel les troisièmes éléments d'assujettissement ou les quatrièmes éléments d'assujettissement sont constitués les uns par des deuxièmes dents en saillie (26) s'étendant parallèlement à un axe de vissage du premier couvercle (5) au récipient (4), et les autres par des deuxièmes logements (27) correspondants conformés pour correspondre aux dents en saillie.

8. Le dispositif médical selon l'une quelconque des revendications précédentes, dans lequel tout ce qui est présent à l'intérieur du boîtier principal extérieur (2) est stérile.
